# FASCICULE DE BREVET EUROPEEN

(11) **EP 1 773 738 B1**
(45) Date de publication et mention de la délivrance du brevet: **02.06.2010**
(21) Numéro de dépôt: 05783841.9
(22) Date de dépôt: 27.06.2005
(51) Int. Cl.: C07C 6/04

(54) **PROCEDE DE TRANSFORMATION D'ETHYLENE EN PROPYLENE**
VERFAHREN ZUR UMWANDLUNG VON ETHYLEN IN PROPYLEN
METHOD FOR CONVERTING ETHYLENE INTO PROPYLENE

(30) Priorité: 02.07.2004 FR 0407395
(43) Date de publication de la demande: 18.04.2007
(73) Titulaire: CPE Lyon Formation Continue et Recherche - CPE Lyon FCR, 69100 Villeurbanne (FR)
(72) Inventeur: COPERET, Christophe, F-69003 LYON (FR); BASSET, Jean-Marie, F-69300 CALUIRE (FR); THIVOLLE-CAZAT, Jean, F-69270 FONTAINES S/ SAONE (FR); LE ROUX, Erwan, 5007 Bergen (NO); TAOUFIK, Mostafa, F-69140 RILLIEUX LA PAPE (FR)
(74) Mandataire: Colombet, Alain André
(86) Numéro de dépôt international: PCT/FR2005/001621
(87) Numéro de publication internationale: WO 2006/013251

(56) Documents cités:
- US-B1- 6 229 060
- US-B1- 6 469 225
- O'NEILL, ROONEY: "Direct transformation of ethylene and propylene on an olefin metathesis catalyst" JOURNAL OF THE AMERICAN CHEMICAL SOCIETY., vol. 94, no. 12, 1972, pages 4383-4384, XP002304625 XXXX cité dans la demande

## Description

La présente invention concerne un procédé de transformation de l'éthylène en propylène.

On observe actuellement une croissance de la demande mondiale en propylène. La méthode classique de production du propylène est la déshydrogénation du propane, qui est basée sur la conversion catalytique endothermique du propane en propylène à des températures atteignant 650 °C. Cette industrie se heurte à deux types de difficultés. La première est d'ordre géographique, les ressources en propane ne se superposant pas forcément aux régions ayant de forts besoins en propylène. L'autre raison est d'ordre économique, le propane étant souvent plus coûteux que le propylène et le procédé lui-même coûteux dans sa mise en oeuvre, notamment en termes de dépense énergétique. D'ailleurs, traditionnellement, les productions de propylène par déshydrogénation du propane interviennent lorsqu'il est possible de s'approvisionner en propane à bas coût et bien souvent le propylène est un simple produit secondaire de l'industrie pétrolière. On voit aujourd'hui apparaître également la production de propylène par métathèse, technologie dans laquelle l'éthylène et le butylène sont combinés pour la production de propylène. Aujourd'hui, selon Alexander H. Tullo, C&EN Northeast News Bureau, 15 décembre 2003; la fabrication en première intention de propylène par déshydrogénation et métathèse ne représente que 2 ou 3 % de la production globale. Environ 69 % du propylène est obtenu comme produit secondaire dans des installations de craquage d'éthylène en présence de vapeur d'eau (« ethylene steam cracker »), le reste provenant d'installations de craquage en lit fluidisé pour produire des carburants (« gasoline-making fluidized catalytic crackers FCCs »). Il serait donc intéressant de disposer d'un nouveau procédé de production de propylène indépendant du propane.

La transformation de l'éthylène en propylène a été annoncée en 1972 par P.P. O'Neill et J.J. Rooney au contact de catalyseur Mo(CO)₆ déposé sur Al₂O₃ sans performance précise (J. Amer. Chem. Soc. 1972, 94, 4383-4). Plus tard, elle a été décrite par H. Imamura et T. Konishi en présence de catalyseurs à base d'europium ou d'ytterbium déposés sur noir (Lanthanide and Actinide Research 1991, 3, 387-9) avec des performances très limitées.

La présente invention a pour objectif de proposer un procédé de transformation de l'éthylène en propylène ayant des performances supérieures à ce qui avait été obtenu par le passé.

Un autre objectif de l'invention est de proposer un tel procédé qui puisse fonctionner à température modérée.

L'invention a donc pour objet un procédé de transformation de l'éthylène en propylène, dans lequel on fait réagir l'éthylène avec un composé métallique supporté comprenant un hydrure de tungstène greffé sur un support à base d'oxyde d'aluminium.

La réaction peut se résumer ainsi :

3 CH₂=CH₂ → 2 CH₂=CH-CH₃

Sans vouloir être lié par la théorie, on pense que la reaction implique au moins en partie une première étape de dimérisation de l'éthylène en butène, puis une réaction de l'éthylène et de butène conduisant au propène.

Le procédé de l'invention peut être conduit à une température allant de 20 à 600 °C. Suivant une caractéristique avantageuse de l'invention, le procédé est conduit à une température relativement basse, allant de 20 à 350 °C, de préférence de 50 à 300 °C, mieux encore de 80 à 200 °C.

Suivant un autre aspect de l'invention, le procédé est conduit sous une pression absolue allant de 0,01 à 8 MPa, de préférence de 0,01 à 1 MPa, mieux encore de 0,1 à 0,5 MPa.

Le procédé selon l'invention peut être conduit en batch dans un réacteur statique. Il est cependant de préférence conduit en continu dans un réacteur dynamique, l'éthylène y étant introduit en continu. La vitesse volumique horaire, c'est-à-dire le volume d'éthylène sur le volume de lit catalytique par unité de temps, peut varier notamment de 4 à 4000 h⁻¹.

Suivant une première modalité, l'éthylène est admis sous forme gazeuse dans le réacteur.

Suivant une deuxième modalité, l'éthylène est admis sous forme liquide dans le réacteur dans lequel il se vaporise.

Suivant un aspect particulier de l'invention, l'éthylène se trouve à l'état résiduel dans un mélange de gaz inerte ou dans un gaz inerte. Le procédé selon l'invention permet de transformer l'éthylène en propylène, le propylène étant plus facile à séparer et éliminer, ce grâce à quoi le procédé de l'invention peut être utilisé pour l'élimination de l'éthylène d'un tel mélange.

Le procédé peut éventuellement être conduit en présence d'hydrogène ou d'un agent formant *in situ* de l'hydrogène. Ainsi le procédé peut être conduit sous une pression partielle d'hydrogène allant de 0,001 à 0,1 MPa. Comme agent formant *in situ* de l'hydrogène, on peut citer les composés cycliques tels que cyclohexane, décahydronaphtalène et tétrahydronaphtalène.

Le catalyseur peut être réactivé ou régénéré par mise en contact avec de l'hydrogène, notamment pur ou dilué dans un gaz neutre. On peut travailler avec une pression d'hydrogène allant de 0,01 à 10 MPa, de préférence de 0,1 à 2 MPa. La régénération peut être conduite de la manière suivante. L'alimentation du réacteur en éthylène est interrompue. On injecte ensuite l'hydrogène et l'on maintien une pression en hydrogène le temps nécessaire à la régénération. Avant de reprendre l'alimentation en éthylène, on peut chasser l'hydrogène en excès par un gaz inerte, e.g. de l'argon. La régénération peut être conduite à une température allant de 50 à 300 °C, notamment de 80 à 200 °C.

Le composé métallique supporté comprend un support à base d'oxyde d' aluminium sur lequel est greffé un hydrure de tungstène. Par hydrure de tungstène greffé sur un support à base d'oxyde d'aluminium, on entend généralement un atome de tungstène lié à au moins un atome d'hydrogène et, notamment par au moins une liaison simple, audit support.

Le composé selon l'invention comprend essentiellement un hydrure de tungstène greffé sur un support à base d'oxyde d'aluminium. Dans ce composé, le support peut être tout support à base d'oxyde d'aluminium, et plus particulièrement tout support où l'oxyde d'aluminium est accessible notamment en surface dudit support. Ainsi le support peut être choisi parmi les supports relativement homogènes en composition à base d'oxyde d'aluminium, ayant notamment une composition à base d'oxyde d'aluminium relativement homogène à travers toute la masse du support, c'est-à-dire depuis le coeur jusqu'à la surface du support, et également parmi les supports hétérogènes à base d'oxyde d'aluminium comprenant de l'oxyde d'aluminium essentiellement en surface des supports. Dans le cas d'un support hétérogène, le support peut comprendre de l'oxyde d'aluminium déposé, supporté ou greffé sur un solide minéral qui peut être lui-même un support solide inorganique, notamment choisi parmi les métaux, les oxydes ou sulfures et les sels, par exemple parmi la silice et les oxydes métalliques.

Le support peut avoir une surface spécifique (B.E.T) choisie dans une gamme allant de 0,1 à 1000 m²/g, de préférence de 0,5 à 800 m²/g. La surface spécifique (B.E.T.) est mesurée selon la norme ISO 9277 (1995).

Le support peut en particulier comprendre de l'oxyde d'aluminium, des oxydes mixtes d'aluminium ou des oxydes d'aluminium modifiés, notamment par un ou plusieurs éléments des Groupes 15 à 17 du Tableau de la Classification Périodique des Eléments .

Par oxyde d'aluminium (encore appelé alumine simple), on entend généralement un oxyde d'aluminium substantiellement exempt de tout autre oxyde (ou contenant moins de 2 % en poids d'un ou plusieurs autres oxydes, présents sous forme d'impuretés). S'il contient plus de 2 % en poids d'un ou plusieurs autres oxydes, alors il est généralement convenu de considérer l'oxyde comme un oxyde mixte d'aluminium, c'est-à-dire un oxyde d'aluminium combiné avec au moins un autre oxyde.

Le support peut de préférence comprendre de l'oxyde d'aluminium choisi parmi les alumines poreuses, les alumines non-poreuses et les alumines mésoporeuses.

Les alumines poreuses sont souvent appelées « alumines activées » ou encore « alumines de transition ». Elles correspondent généralement à différents oxydes d'aluminium, Al₂O₃, partiellement hydroxylés. Il s'agit de supports poreux obtenus généralement par un traitement dit d'« activation » comprenant notamment un traitement thermique (ou de déshydratation) d'un précurseur choisis parmi les hydroxydes d'aluminium, tels que les tri-hydroxydes d'aluminium, les hydroxydes de l'oxyde d'aluminium ou les hydroxydes gélatineux d'aluminium. Le traitement d'activation permet d'éliminer l'eau contenue dans le précurseur, mais aussi en partie les groupes hydroxyle, laissant ainsi subsister quelques groupes hydroxyle résiduels et une structure poreuse spécifique. La surface des alumines poreuses comprend généralement un mélange complexe d'atomes d'aluminium et d'oxygène et d'ions hydroxyle qui se combinent selon des formes cristallines spécifiques et qui notamment produisent à la fois des sites acides et basiques. On peut ainsi choisir comme support solide une alumine poreuse parmi l'alumine-γ (alumine-gamma), l'alumine-η (alumine-éta), l'alumine-δ (alumine-delta), l'alumine-θ (alumine-théta), l'alumine-κ (alumine-kappa), l'alumine-ρ (alumine-ro) et l'alumine-χ (alumine-ksi ou -chi), et de préférence parmi l'alumine-γ et l'alumine-η. Ces différentes formes cristallines dépendent essentiellement du choix du précurseur et des conditions du traitement d'activation, en particulier la température et la pression. Le traitement d'activation peut être réalisé par exemple sous un courant d'air ou un courant d'un autre gaz, notamment un gaz inerte, à une température pouvant être choisie dans une gamme allant de 100 à 1000°C, de préférence de 200 à 1000°C.

On peut également utiliser des alumines poreuses ou encore semi-poreuses, préparées par un traitement d'activation comme précédemment décrit, notamment à une température allant de 600 à 1000°C. Ces alumines poreuses ou semi-poreuses peuvent comprendre des mélanges d'alumines poreuses sous l'une au moins des formes cristallines précédemment décrites, telles que l'alumine-γ, l'alumine-η, l'alumine-δ, l'alumine-θ, l'alumine-κ, l'alumine-ρ ou l'alumine-χ, avec une alumine non-poreuse, en particulier l'alumine-α, notamment en une proportion de 20 à 80 % en poids.

Les alumines poreuses sont généralement des produits de décomposition thermique des tri-hydroxydes d'aluminium, des hydroxydes de l'oxyde d'aluminium (ou hydrates de l'oxyde d'aluminium) et des hydroxydes gélatineux d'aluminium (ou gels d'alumine).

Les tri-hydroxydes d'aluminium de formule générale Al(OH)₃ = Al₂O₃, 3H₂O peuvent exister sous différentes formes cristallines, telles que la gibbsite ou l'hydrargillite (Al(OH)₃ -α), la bayérite (Al(OH)₃-β), ou la nordstrandite. Les tri-hydroxydes d'aluminium peuvent être obtenus par précipitation à partir de sels d'aluminium dans des solutions généralement alcalines.

Les hydroxydes de l'oxyde d'aluminium de formule générale AlO(OH) = Al₂O₃, H₂O peuvent également exister sous différentes formes cristallines, telles que le diaspore (AlO(OH)-β) ou la boehmite (ou AlO(OH)-α). Le diaspore peut se trouver dans certains types d'argile et de bauxite, et peut être synthétisé par un traitement thermique de la gibbsite à environ 150°C, ou par un traitement hydrothermique de la boehmite à 380°C sous une pression de 50MPa. La boehmite peut être facilement obtenue en chauffant le précipité gélatineux formé en traitant à froid des solutions de sels d'aluminium avec de l'ammoniac. Les hydroxydes de l'oxyde d'aluminium peuvent être aussi obtenus par hydrolyse d'alcoolates d'aluminium.

Les hydroxydes gélatineux d'aluminium (ou gels d'alumine) sont généralement des polyhydroxydes d'aluminium, notamment de formule générale :

nAl(OH)₃, (n-1)H₂O (1)

dans laquelle n est un nombre variant de 1 à 8. Les hydroxydes gélatineux d'aluminium peuvent être obtenus par l'un des procédés choisis parmi la décomposition thermique d'un sel d'aluminium, tel que le chlorure d'aluminium, l'électrolyse de sels d'aluminium, tels qu'un mélange de sulfate d'aluminium et de sulfate alcalin, l'hydrolyse d'alcoolates d'aluminium, tels que le méthylate d'aluminium, la précipitation à partir d'aluminates, tels que des aluminates alcalins ou alcalino-terreux, et la précipitation à partir de sels d'aluminium, par exemple par mise en contact de solutions aqueuses de Al₂(SO₄)₃ et d'ammoniac, ou de NaAlO₂ et d'un acide, ou de NaAlO₂ et de Al₂(SO₄)₃, les précipités ainsi obtenus pouvant subir ensuite un vieillissement et un séchage pour éliminer l'eau. Les hydroxydes gélatineux d'aluminium se présentent généralement sous la forme d'un gel d'alumine amorphe, notamment sous la forme d'une pseudoboehmite.

Les alumines poreuses peuvent avoir une surface spécifique (B.E.T.) choisie dans une gamme allant de 100 à 1000 m²/g, de préférence de 300 à 1000 m²/g, notamment de 300 à 800 m²/g, en particulier de 300 à 600 m²/g. Elles peuvent en outre présenter un volume spécifique de pore égal ou inférieur à 1 cm³/g, de préférence égal ou inférieur à 0,9 cm³/g, notamment égal ou inférieur à 0,6 cm³/g.

Le support peut aussi comprendre des alumines non-poreuses, de préférence l'alumine-α (alumine-alpha), connue généralement sous le terme de d'« alumine calcinée » ou d'« alumine de flamme». L'alumine-α existe à l'état naturel sous le terme de «corindon ». Elle peut être synthétisée généralement par un traitement thermique ou une calcination d'un précurseur choisi notamment parmi les sels de l'aluminium, les hydroxydes de l'oxyde d'aluminium, les tri-hydroxydes d'aluminium et les oxydes d'aluminium, tels que l'alumine-γ, à une température supérieure à 1000°C, de préférence supérieure à 1100°C. Elle peut contenir des impuretés telles que d'autres oxydes, par exemple Fe₂O₃, SiO₂, TiO₂, CaO, Na₂O, K₂O, MgO, SrO, BaO et Li₂O, en des proportions inférieures à 2 %, de préférence à 1 % en poids. Les alumines non-poreuses, telles que l'alumine-α, peuvent avoir une surface spécifique (B.E.T.) choisie dans une gamme allant de 0,1 à moins de 300 m²/g, de préférence de 0,5 à 300 m²/g, notamment de 0,5 à 250 m²/g.

Le support peut également comprendre des alumines mésoporeuses, ayant notamment une surface spécifique (B.E.T.) choisie dans une gamme allant de 100 à 800 m²/g. Les alumines mésoporeuses ont généralement des pores d'une largeur allanr de 2 nm à 0,05 µm.

Le support peut aussi comprendre des oxydes mixtes d'aluminium. Par oxydes mixtes d'aluminium, on entend généralement des oxydes d'aluminium combinés avec au moins un autre oxyde en une proportion pondérale de préférence de 2 à moins de 80 %, notamment de 2 à moins de 50 %, en particulier de 2 à moins de 40 % ou même de 2 à moins de 30 %. Le ou les autres oxydes peuvent être des oxydes des éléments, M, choisis parmi les métaux des Groupes 1 à 13 et les éléments du Groupe 14, à l'exception du carbone, du Tableau de la Classification Périodique des Eléments. Plus particulièrement, ils peuvent être des oxydes des éléments M choisis parmi les métaux alcalins, les métaux alcalino-terreux, les métaux de transition et les éléments des Groupes 13 et 14 dudit Tableau, à l'exception du carbone. Les métaux de transition comprennent généralement les métaux des Groupes 3 à 11 dudit Tableau, en particulier les éléments 21 à 29, 39 à 47, 57 à 79 (incluant les lanthanides) et les actinides. Le ou les autres oxydes des éléments M sont de préférence choisis parmi les métaux de transition des Groupes 3 à 7, les lanthanides, les actinides et les éléments des Groupes 13 et 14 dudit Tableau, à l'exception du carbone. Plus particulièrement ils peuvent être choisis parmi les oxydes de silicium, de bore, de gallium, de germanium, de titane, de zirconium, de cérium, de vanadium, de niobium, de tantale, de chrome, de molybdène et de tungstène.

On peut choisir les oxydes mixtes d'aluminium parmi les aluminates anhydres, les spinelles et les aluminosilicates. En particulier, on peut choisir les aluminates anhydres parmi les aluminates alcalins anhydres, tels que l'aluminate de lithium anhydre (LiAlO₂) ou l'aluminate de sodium anhydre (Na₂O, Al₂O₃), et les aluminates alcalino-terreux anhydres, tels que l'aluminate tricalcique anhydre (3CaO, Al₂O₃) ou l'aluminate de béryllium anhydre (BeO, Al₂O₃). On peut choisir en particulier les spinelles parmi les oxydes d'aluminium combinés avec les oxydes des métaux divalents, et en particulier parmi le spinelle de magnésium (MgAl₂O₄), le spinelle de calcium (CaAl₂O₄), le spinelle de zinc (ZnAl₂O₄), le spinelle de manganèse (MnAl₂O₄); le spinelle de fer (FeAl₂O₄) et le spinelle de cobalt (CoAl₂O₄). On peut choisir en particulier les aluminosilicates parmi les argiles, le talc, les micas, le feldspath, les aluminosilicates micro-poreux, notamment les tamis moléculaires, et les zéolites.

Le support peut également comprendre des oxydes d'aluminium modifiés, notamment modifiés par un ou plusieurs éléments des Groupes 15 à 17, de préférence des Groupes 16 à 17 du Tableau de la Classification Périodique des Eléments, par exemple le phosphore, le soufre, le fluor ou le chlore. Le support peut notamment comprendre les super-acides d'alumine ou les oxydes d'aluminium sulfatés, sulfurés, chlorés ou fluorés. La classification utilisée est celle définie par l'IUPAC en 1991 dans lequel les groupes sont numérotés de 1 à 18 et que l'on trouve par exemple dans « CRC Handbook of Chemistry and Physics », 76th Edition (1995-1996), de David R. Lide, publié par CRC Press, Inc. USA.

Le support peut être un support homogène en composition notamment à travers toute la masse du support. Il peut être aussi un support hétérogène à base d'oxyde d'aluminium, support dans lequel l'oxyde d'aluminium, les oxydes mixtes d'aluminium ou les oxydes d'aluminium modifiés, tels que décrits précédemment, sont essentiellement disposés en surface du support, et le coeur du support est essentiellement constitué par un solide minéral choisi notamment parmi les métaux, les oxydes ou sulfures, et les sels, tels que la silice ou les oxydes métalliques. Le support hétérogène peut être préparé par dispersion, par précipitation et/ou par greffage sur le solide minéral d'un des précurseurs des composés à base d'oxyde d'aluminium cités précédemment. Les précurseurs peuvent être notamment choisis parmi les hydroxydes d'aluminium, en particulier parmi les tri-hydroxydes d'aluminium, les hydroxydes de l'oxyde d'aluminium et les hydroxydes gélatineux d'aluminium. On préfère les hydroxydes gélatineux d'aluminium, tels que décrits précédemment, connus sous le terme de gels d'alumine ou d'alumines amorphes. La préparation d'un support hétérogène peut être réalisée notamment en mettant en oeuvre un tel précurseur par voie d'un sol-gel ou à l'aide d'un composé organométallique qui facilite en particulier le greffage sur le solide minéral.

Le composé selon l'invention se présente généralement sous forme de particules qui peuvent avoir toute forme et toute taille, notamment une taille moyenne allant de 10 nm à 5 mm, de préférence de 20 nm à 4mm. Les particules du support peuvent se présenter telles quelles ou peuvent être mises en forme de façon à avoir une forme spécifique, notamment une forme sphérique, sphéroïdale, hémisphérique, hémisphéroïdale, cylindrique ou cubique, ou une forme d'anneaux, de pastilles, de disques ou de granulés.

Le composé selon l'invention comprend essentiellement un hydrure de tungstène greffé le support à base d'oxyde d'aluminium. Le degré d'oxydation du tungstène dans le composé métallique supporté peut avoir une valeur choisie dans une gamme allant de 2 à 6, de préférence de 4 à 6. L'atome de tungstène est lié notamment au support solide, notamment par au moins une liaison simple. Il peut être en outre lié à un ou plusieurs atomes d'hydrogène par des liaisons simples (W - H) et éventuellement à un ou plusieurs radicaux hydrocarbonés, R, notamment par des liaisons carbone-tungstène simples ou multiples. Le nombre d'atomes d'hydrogène liés à un atome de tungstène dépend du degré d'oxydation du tungstène, du nombre de liaisons simples liant ledit atome de tungstène au support et éventuellement du nombre de liaisons simples ou multiples liant ledit atome de tungstène au radical hydrocarboné, R. Ainsi, le nombre d'atomes d'hydrogène liés à un atome de tungstène peut être au moins égal à 1 et au plus égal à 5, et peut aller de préférence de 1 à 4, de préférence de 1 à 3. Par greffage de l'hydrure de tungstène au support solide à base d'oxyde d'aluminium, on entend généralement que l'atome de tungstène est lié par au moins une liaison simple audit support, et plus particulièrement par au moins une liaison simple (W-OAl) à au moins un atome d'oxygène de l'oxyde d'aluminium. Le nombre de liaisons simples liant l'atome de tungstène au support, notamment par une liaison simple (W-OAl), dépend du degré d'oxydation du tungstène et du nombre des autres liaisons liant l'atome de tungstène, et est généralement égal à 1, 2 ou 3.

L'atome de tungstène du composé selon l'invention peut être éventuellement lié à un ou plusieurs radicaux hydrocarbonés, R, par une ou plusieurs liaisons carbone-tungstène simples, doubles ou triples. Le ou les radicaux hydrocarbonés, R, peuvent être des radicaux hydrocarbonés, identiques ou différents, saturés ou insaturés, en particulier comprenant de 1 à 20, de préférence de 1 à 10 atomes de carbone, et comprenant éventuellement du silicium, notamment dans un groupement organo-silane. Ils peuvent être choisis notamment parmi les radicaux alkyle, notamment linéaires ou ramifiés, aliphatiques ou alicycliques, par exemple les radicaux alkyle, alkylidène ou alkylidyne, notamment de C₁ à C₁₀, parmi les radicaux aryle, notamment de C₆ à C₁₂, et parmi les radicaux aralkyle, aralkylidène ou aralkylidyne, notamment de C₇ à C_{14.}

L'atome de tungstène de l'hydrure de tungstène greffé peut être lié au radical hydrocarboné, R, par une ou plusieurs liaisons carbone-tungstène simples, doubles ou triples. Il peut s'agir d'une liaison carbone-tungstène simple, notamment de type σ : dans ce cas, le radical hydrocarboné, R, peut être un radical alkyle, notamment linéaire ou ramifié, ou un radical aryle, par exemple le radical phényle, ou un radical aralkyle, par exemple le radical benzyle ou le radical de formule (C6H5-CH2-CH2-). Par radical alkyle, on entend généralement un radical monovalent aliphatique provenant de l'enlèvement d'un atome d'hydrogène sur un atome de carbone de la molécule d'un alcane, ou d'un alcène, ou d'un alcyne, ou même d'un organo-silane, par exemple un radical méthyle (CH3-), éthyle (C2H5-), propyle (C2H5-CH2-), néopentyle ((CH3)3C-CH2-), allyle (CH2=CH-CH2-), alkynyle (R-C ≡ C-), notamment éthynyle (CH ≡ C-), ou néosilyle ((CH₃)₃Si-CH₂ - ). Le radical alkyle peut être par exemple de formule (R'-CH2-) où R' représente un radical alkyle, linéaire ou ramifié.

Il peut aussi s'agir d'une liaison carbone-tungstène double, notamment de type π : dans ce cas, le radical hydrocarboné, R, peut être un radical alkylidène, notamment linéaire ou ramifié, ou un radical aralkylidène. Par radical alkylidène, on entend généralement un radical bivalent aliphatique provenant de l'enlèvement de deux atomes d'hydrogène sur un même atome de carbone de la molécule d'un alcane, ou d'un alcène, ou d'un alcyne, ou même d'un organo-silane, par exemple un radical méthylidène (CH2=), éthylidène (CH3-CH=), propylidène (C2H5-CH=), néopentylidène ((CH3)3C-CH=), ou allylidène (CH2=CH-CH=). Le radical alkylidène peut être par exemple de formule (R'-CH=) où R' représente un radical alkyle, linéaire ou ramifié. Par radical aralkylidène, on entend généralement un radical bivalent aliphatique provenant de l'enlèvement de deux atomes d'hydrogène sur un même carbone d'un radical alkyle, alcényle ou alcynyle branché sur un groupement aromatique.

Il peut également s'agir d'une liaison carbone-tungstène triple : dans ce cas, le radical hydrocarboné, R, peut être un radical alkylidyne, notamment linéaire ou ramifié, ou un radical aralkylidyne. Par radical alkylidyne, on entend généralement un radical trivalent aliphatique provenant de l'enlèvement de trois atomes d'hydrogène sur un même atome de carbone de la molécule d'un alcane, ou d'un alcène, ou d'un alcyne, ou même d'un organo-silane, par exemple un radical éthylidyne (CH3-C≡), propylidyne (C2H5-C≡), néopentylidyne ((CH3)3C-C≡), ou allylidyne (CH2=CH-C≡). Le radical alkylidyne peut être par exemple de formule (R'-C≡) où R' représente un radical alkyle, linéaire ou ramifié. Par radical aralkylidyne, on entend généralement un radical trivalent aliphatique provenant de l'enlèvement de trois atomes d'hydrogène sur un même carbone d'un radical alkyle, alcényle ou alcynyle branché sur un groupement aromatique.

Plus particulièrement, le radical hydrocarboné, R, peut être choisi parmi les radicaux méthyle, éthyle, n-propyle, isopropyle, n-butyle, isobutyle, néopentyle, allyle, néopentylidène, allylidène, néopentylidyne et néosilyle.

L'atome de tungstène du composé selon l'invention peut être complexé par un ou plusieurs ligands hydrocarbonés, en particulier des ligands aromatiques ou carbonyle.

On peut représenter schématiquement l'hydrure de tungstène greffé sur le support à base d'oxyde d'aluminium par la formule suivante : 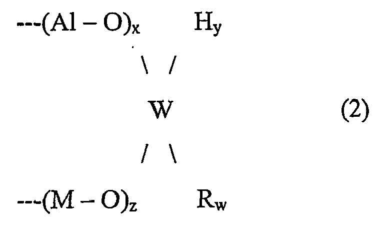 dans laquelle W, Al, O et H représentent respectivement des atomes de tungstène, d'aluminium, d'oxygène et d'hydrogène, M représente un atome d'un ou plusieurs éléments d'un autre oxyde, tels que définis précédemment, R représente un radical hydrocarboné, tel que défini précédemment, et x, y, w et z sont des nombres entiers dont la somme (w + x + y + z) = 2 à 6, et avec x = 1 à 3, y = 1 à 5, w = 0 à 4 et z = 0 à 2. Dans la formule (2), les liaisons ---(Al - O) et ---(M - O) représentent une ou plusieurs liaisons simples ou multiples reliant respectivement l'atome d'aluminium et l'atome M à l'un des constituants atomiques du support à base d'oxyde d'aluminium, notamment à l'un des atomes d'oxygène de ce support.

Le composé selon l'invention présente généralement en spectroscopie infra-rouge une ou plusieurs bandes d'absorption spécifiques de la liaison (W - H), bandes dont la fréquence peut varier selon la sphère de coordination du tungstène et dépendre notamment du nombre de liaisons du tungstène avec le support, avec les radicaux hydrocarbonés R et avec d'autres atomes d'hydrogène. Ainsi on a par exemple trouvé au moins des deux bandes d'absorption à 1903 et 1804 cm⁻¹, bandes spécifiques en particulier de la liaison (W-H) considérée notamment dans l'environnement des liaisons (W-OAl) liant le même atome de tungstène à un atome d'oxygène lié lui-même à un atome d'aluminium d'une alumine-α. A titre de comparaison, l'hydrure de tungstène greffé dans les mêmes conditions sur un support de silice présente généralement en spectroscopie infra-rouge une au moins des deux bandes d'absorption à 1940 et 1960 cm⁻¹, bandes qui sont différentes des précédentes et qui sont en particulier spécifiques de la liaison (W-H) considérée notamment dans l'environnement des liaisons (W-OSi) liant le même atome de tungstène à un atome d'oxygène lié lui-même à un atome de silicium du support de silice.

Une autre façon pouvant caractériser la présence d'une liaison (W - H) dans le composé selon l'invention vient d'une mesure par Résonance Magnétique Nucléaire du proton (¹H-RMN solide) sous 500 MHz où la valeur du déplacement chimique de l'hydrure de tungstène (δ _{W-H}) est égale à 10,6 ppm (parties par million).

Le composé selon l'invention peut en outre comprendre un hydrure d'aluminium, notamment en surface du support et en particulier au voisinage de l'hydrure de tungstène greffé. On pense qu'un hydrure d'aluminium peut se former par ouverture d'un pont aluminoxane (de formule Al-O-Al) présent notamment en surface du support et par réaction entre un atome d'hydrogène d'un hydrure de tungstène greffé et le pont aluminoxane ainsi ouvert. Un test simple de caractérisation de l'hydrure d'aluminium présent dans le composé de l'invention à côté d'un hydrure de tungstène comprend une réaction de deutériation dudit composé. Le test peut être réalisé par mise en contact du composé selon l'invention avec une atmosphère de deutérium sous une pression absolue de 66,7 kPa, à une température choisie entre 25 et 80°C, de préférence égale à 60°C, pendant une durée de 15 minutes. Une réaction sélective de deutériation est ainsi réalisée dans ces conditions : elle permet de substituer les atomes d'hydrogène par des atomes de deutérium dans les liaisons (W-H) et former ainsi de nouvelles liaisons (W - D) qui en spectroscopie infra-rouge présentent deux bandes d'absorption à 1293 et 1393 cm⁻¹, tout en laissant inchangés les atomes d'hydrogène dans les liaisons (Al-H) que l'on peut alors caractériser en spectroscopie infra-rouge par une bande d'absorption à 1914 cm⁻¹.

La présente invention concerne également un procédé de préparation du composé métallique supporté. Le composé selon l'invention qui se présente essentiellement sous la forme d'un hydrure de tungstène greffé sur un support à base d'oxyde d'aluminium, peut être préparé par un procédé comprenant les étapes suivantes :
(1) une étape de dispersion et de greffage d'un précurseur organométallique de tungstène (Pr) sur un support à base d'oxyde d'aluminium, précurseur dans lequel le tungstène est notamment lié ou complexé à au moins un ligand hydrocarboné, de façon à former un composé ou complexe hydrocarboné de tungstène greffé sur ledit support, puis
(2) une étape d'hydrogénolyse du composé ou complexe hydrocarboné de tungstène greffé, résultant de l'étape précédente, de façon à former un hydrure de tungstène greffé sur ledit support.

Le précurseur organométallique de tungstène, Pr, comporte de préférence un atome de tungstène lié ou complexé à un ou plusieurs ligands hydrocarbonés. L'atome de tungstène peut être notamment lié à un carbone du ligand hydrocarboné par des liaisons (carbone-tungstène) simples, doubles ou triples. Les ligands hydrocarbonés peuvent être des radicaux hydrocarbonés, identiques ou différents, saturés ou insaturés, notamment aliphatiques ou alicycliques, de préférence de C₁ à C₂₀, en particulier de C₁ à C₁₀, et peuvent être choisis notamment parmi les radicaux hydrocarbonés, R, décrits précédemment. Le nombre de ligands hydrocarbonés liés à l'atome de tungstène dépend du degré d'oxydation du tungstène dans le précurseur Pr et peut être au plus égal au degré d'oxydation du tungstène dans le précurseur Pr, en particulier être supérieur à 0 et au plus égal au degré d'oxydation maximal du tungstène et de préférence avoir toute valeur allant de 2 à 6, notamment de 4 à 6.

Le précurseur Pr peut comporter un atome de tungstène complexé à un ou plusieurs ligands hydrocarbonés tels que le degré d'oxydation du tungstène soit égal à zéro. Le ligand hydrocarboné peut être choisi parmi les ligands aromatiques ou les ligands carbonyle. Ainsi, le précurseur Pr peut être choisi parmi le tungstène bis-arène et le tungstène hexacarbonyle.

Préalablement à la première étape de dispersion et de greffage, le support à base d'oxyde d'aluminium peut être soumis à une étape préalable de calcination et/ou de déshydroxylation. La calcination du support peut être réalisée de façon à oxyder le carbone éventuellement présent dans le support et à l'éliminer sous forme de dioxyde de carbone. Elle peut être réalisée en soumettant le support à un traitement thermique oxydant, notamment sous un courant d'air sec, à une température inférieure à la température de frittage du support, par exemple à une température allant de 100 à 1000°C, de préférence de 200 à 800°C, pendant une durée suffisante permettant d'éliminer le dioxyde de carbone et pouvant aller de 0,1 à 48 heures, sous une pression inférieure, égale ou supérieure à la pression atmosphérique.

Le support peut également être soumis à une autre étape préalable, dite de déshydroxylation. Cette étape peut être réalisée de façon à éliminer éventuellement l'eau résiduelle du support et une partie des groupes hydroxyle, à laisser subsister notamment en surface du support une quantité résiduelle des groupes hydroxyle et à former éventuellement des ponts aluminoxane (de formule Al-O-Al). La déshydroxylation peut être réalisée en soumettant le support à un traitement thermique sous un courant gazeux inerte, par exemple sous un courant d'azote, d'argon ou d'hélium, sous une pression de préférence inférieure à la pression atmosphérique, par exemple sous une pression absolue allant de 10⁻⁴ Pa à 10² kPa, de préférence de 10⁻² Pa à 50 kPa, à une température inférieure à la température de frittage du support, par exemple à une température allant de100 à 1000°C, de préférence de 200 à 800°C, et pendant une durée suffisante permettant de laisser une quantité résiduelle appropriée de groupes hydroxyle et/ou aluminoxane dans le support et pouvant aller de 0,1 à 48 heures. L'étape de déshydroxylation peut être avantageusement réalisée après l'étape de calcination.

L'étape de dispersion et de greffage peut être réalisée par sublimation, par imprégnation à l'aide d'un solvant, ou par mélange à sec. Dans le cas d'une étape par sublimation, le précurseur Pr qui se présente généralement à l'état solide dans les conditions normales, est chauffé notamment sous une pression inférieure à la pression atmosphérique et dans des conditions de température assurant sa sublimation et sa migration à l'état gazeux sur le support. La sublimation peut être réalisée à une température allant de - 30 à 200°C, et notamment sous une pression absolue allant de 10⁻⁴ à 1 Pa. Le greffage du précurseur Pr sur le support peut être contrôlé par spectroscopie infra-rouge. L'excès de précurseur Pr qui ne s'est pas greffé sur le support, peut être éliminé par sublimation inverse.

L'étape de dispersion et de greffage peut aussi être réalisée par imprégnation à l'aide d'un solvant. Dans ce cas, le précurseur Pr peut être mis en solution dans un solvant organique, polaire ou non polaire, par exemple le pentane ou l'éther éthylique. L'imprégnation peut être réalisée par mise en contact du support à base d'oxyde d'aluminium avec la solution du précurseur Pr préparée précédemment. L'imprégnation peut être réalisée à une température allant de -80 à 200°C, sous une atmosphère inerte, par exemple une atmosphère d'azote, d'argon ou d'hélium, et de préférence sous agitation. On obtient ainsi une suspension d'un composé ou complexe hydrocarboné de tungstène greffé sur le support. On peut éliminer l'excès de précurseur Pr qui ne s'est pas greffé sur le support, par lavage à l'aide d'un solvant organique, identique ou différent de celui utilisé lors de l'imprégnation.

L'étape de dispersion et de greffage peut également être réalisée par mélange à sec, notamment par mélange mécanique à sec, en l'absence de liquide ou de solvant liquide. Dans ce cas, le précurseur Pr qui est présent sous forme d'un solide, est mélangé avec le support à base d'oxyde d'aluminium, en l'absence de liquide ou de solvant liquide, en particulier sous agitation mécanique et sous une atmosphère inerte, par exemple une atmosphère d'azote, d'argon ou d'hélium, de façon à former un mélange de deux solides. Pendant ou après le mélange à sec, on peut réaliser un traitement thermique et/ou un traitement sous une pression inférieure à la pression atmosphérique, de façon à faire migrer et réagir le précurseur Pr avec le support. Le précurseur qui n'a pas été greffé sur le support, peut être éliminé par sublimation inverse ou par lavage à l'aide d'un solvant organique.

La préparation du composé selon l'invention peut comprendre une seconde étape dite d'hydrogénolyse. Il s'agit d'une réaction d'hydrogénolyse du composé ou complexe hydrocarboné de tungstène greffé sur le support, tel que préparé à l'étape précédente. La réaction est généralement réalisée de façon à former un hydrure de tungstène greffé sur le support. Par hydrogénolyse, on entend généralement une réaction de scission d'une molécule avec fixation d'hydrogène sur les deux portions scindées. En l'occurrence, la réaction de scission se produit notamment entre l'atome de tungstène greffé sur le support et l'atome de carbone du précurseur Pr fixé ou complexé avec ledit atome de tungstène. L'hydrogénolyse peut être réalisée à l'aide d'hydrogène ou d'un agent réducteur, capable notamment de transformer le composé ou complexe hydrocarboné de tungstène greffé en hydrure de tungstène greffé. L'hydrogénolyse peut être réalisée en mettant en contact le composé ou complexe hydrocarboné de tungstène greffé avec de l'hydrogène ou l'agent réducteur. Elle peut être réalisée sous une atmosphère d'hydrogène ou une atmosphère inerte lorsqu'on utilise un agent réducteur, sous une pression absolue allant de 10⁻² à 10 MPa, à une température allant de 20 à 500°C, pendant une durée allant de 0,1 à 48 heures.

Les exemples suivants illustrent la présente invention sans la limiter.

### Exemple 1: préparation d'un hydrure de tungstène greffé sur une alumine.

Dans une étape préalable, 530 mg d'une alumine-γ ayant une taille moyenne de 40 µm et une surface spécifique (B.E.T.) de 200 m²/g, contenant 90 % en poids d'alumine et 9 % en poids d'eau, et vendue par Johnson Matthey (Grand-Bretagne), sont soumis à un traitement de calcination sous un courant d'air sec à 500°C pendant 15 heures, puis à un traitement de déshydroxylation sous une pression absolue de 10⁻² Pa, à 500°C pendant 15 heures, de sorte que l'alumine ainsi calcinée et déshydroxylée présente en spectroscopie infra-rouge trois bandes d'absorption respectivement à 3774, 3727 et 3683 cm⁻¹, caractéristiques notamment de liaison (AlO-H) résiduelle.

Dans une première étape, on introduit dans un réacteur en verre, sous atmosphère d'argon et à 25°C, les 530 mg de l'alumine préparée précédemment, puis une solution de 6 ml de n-pentane contenant 300 mg de tris(néopentyl)néopentylidyne tungstène, utilisé comme précurseur Pr et répondant à la formule générale:

W [-CH₂-C(CH₃)₃]₃ [≡ C-C(CH₃)₃] (3)

Le mélange ainsi obtenu est maintenu à 25°C pendant 3 heures. Au bout de ce temps, on obtient un composé organo-métallique de tungstène greffé sur l'alumine, l'excès de précurseur Pr qui n'a pas réagi, étant éliminé par lavage avec du n-pentane à 25°C. Le composé organo-métallique de tungstène ainsi greffé est séché sous vide. Il contient 1,5 % en poids de tungstène et répond à la formule générale:

(Al-O)ₓ W [-CH₂-C(CH₃)₃]_{y} [=CH-C(CH₃)] (4)

avec=1 et y = 2 .

Dans une seconde étape, on isole 50 mg du composé organo-métallique de tungstène greffé obtenu précédemment qui sont soumis dans un réacteur en verre à un traitement d'hydrogénolyse par mise en contact avec de l'hydrogène, sous une pression absolue d'hydrogène de 73 kPa, à 150°C, pendant 15 heures. Au bout de ce temps, on refroidit le réacteur à 25°C, on obtient et on isole sous argon un composé (W/Al-1) selon l'invention qui comprend notamment un hydrure de tungstène greffé sur l'alumine. Le composé (W/Al-1) contient 1,5 % en poids de tungstène et présente en spectroscopie infra-rouge deux bandes d'absorption respectivement à 1903 et 1804 cm⁻¹, caractéristiques de la liaison (W-H) notamment greffée sur l'alumine.

### Exemple 2 : préparation d'un hydrure de tungstène greffé sur une alumine.

Les étapes préalables de calcination et de déshydroxylation de l'alumine-α sont exactement identiques à celles de l'Exemple 1.

Dans une première étape, on isole 53 mg de l'alumine préparée précédemment et on les introduit dans un réacteur en verre à 25°C sous une atmosphère d'argon. Puis, on introduit dans le réacteur le précurseur Pr de formule générale (3) tel qu'utilisé à l'Exemple 1. On chauffe alors le réacteur à 70°C pendant 2 heures, de façon à sublimer le précurseur Pr sur l'alumine et à former un composé organométallique de tungstène greffé sur l'alumine. Au bout de ce temps, on élimine l'excès de précurseur Pr n'ayant pas réagi, par sublimation inverse à 70°C. Ensuite, on refroidit le réacteur à 25°C et on isole sous argon un composé organométallique de tungstène ainsi greffé qui contient 3,7 % en poids de tungstène et qui répond à la formule générale (4) précédente.

La seconde étape est réalisé exactement comme à l'Exemple 1, excepté le fait qu'on utilise le composé organométallique de tungstène greffé sur l'alumine préparé à l'étape précédente. On obtient ainsi un composé (W/Al-2) selon l'invention comprenant un hydrure de tungstène greffé sur l'alumine et contenant 3,7 % en poids de tungstène. Il présente en spectroscopie infra-rouge deux bandes d'absorption respectivement à 1903 et 1804 cm⁻¹, caractéristiques de la liaison (W - H) notamment greffée sur l'alumine.

Le composé (W/Al-2) est soumis à un test sélectif de deutériation montrant qu'il comprend un hydrure de tungstène et un hydrure d'aluminium, tous les deux greffés sur l'alumine. Un échantillon du composé (W/Al-2) est placé dans un réacteur en verre, puis est mis en contact dans ce réacteur avec une atmosphère de deutérium sous une pression absolue de 66,7 kPa, à une température de 60°C, pendant 15 minutes. Au bout de ce temps, on refroidit le réacteur à 25°C et on isole sous argon le composé solide ainsi deutéré qui présente en spectroscopie infra-rouge une bande d'absorption à 1914 cm⁻¹, caractéristique de la liaison (Al - H) inchangée par la réaction de deutériation réalisée dans ces conditions. On observe par ailleurs que les bandes d'absorption à 1903 et 1804 cm⁻¹ caractéristiques de la liaison (W - H) greffée sur l'alumine disparaissent au profit des bandes d'absorption respectivement à 1293 et 1393 cm⁻¹, caractéristiques de la liaison (W - D) greffée sur l'alumine et formée par la réaction de deutériation des liaisons (W - H).

### Exemple 3: préparation d'un hydrure de tungstène greffé sur une alumine.

Les étapes préalables de calcination et de déshydroxylation de l'alumine sont exactement identiques à celles décrit dans l'exemple 1.

Dans une première étape, on isole 2 g de l'alumine préparée précédemment et on les introduit sous atmosphère d'argon dans un réacteur en verre à 25°C muni d'un barreau magnétique d'agitation. Puis dans le réacteur, on introduit 305 mg du précurseur Pr de formule générale (3) tel qu'utilisé à l'Exemple 1. On chauffe le réacteur à 66°C et on agite le mélange à sec ainsi réalisé pendant 4 heures. Au bout de ce temps on refroidit le réacteur à 25°C, puis on lave le mélange de solide avec du n-pentane à 25°C. Le composé solide ainsi lavé est séché sous vide, puis isolé sous argon de façon à obtenir un composé organométallique de tungstène greffé sur l'alumine contenant 3,9 % en poids de tungstène et qui répond à la formule générale (4) précédente.

On réalise la seconde étape exactement comme à l'Exemple 1, excepté le fait qu'on utilise le composé organométallique de tungstène greffé sur l'alumine préparé précédemment. On obtient ainsi un composé (W/Al-3) selon l'invention comprenant un hydrure de tungstène greffé sur l'alumine et contenant 3,9 % en poids de tungstène. Il présente en spectroscopie infra-rouge deux bandes d'absorption respectivement à 1903 et 1804 cm⁻¹, caractéristiques de la liaison (W-H) greffée sur l'alumine. Par ailleurs, il présente en résonance magnétique nucléaire (¹H-RMN solide) sous 500 MHz une valeur du déplacement chimique de l'hydrure de tungstène (δ_{W-H}) égale à 10,6 ppm (parties par million).

### Exemple 4: préparation d'un hydrure de tungstène greffé sur une silice-alumine.

Dans une étape préalable, 530 mg d'une silice-alumine ayant une surface spécifique (B.E.T.) de 475 m²/g, contenant 33 % en poids d'alumine, et vendue par Akzo Nobel, sont soumis à un traitement de calcination sous un courant d'air sec à 500°C pendant 15 heures, puis à un traitement de déshydroxylation sous une pression absolue de 10⁻² Pa, à 500°C pendant 15 heures, de sorte que la silice-alumine ainsi calcinée et déshydroxylée présente en spectroscopie infra-rouge une bande d'absorption à 3747 cm⁻¹, caractéristique notamment de liaison (SiO-H) résiduelle.

Dans une première étape, on introduit dans un réacteur en verre, sous atmosphère d'argon et à 25°C, les 530 mg de silice-alumine préparée précédemment, puis une solution de 6 ml de n-pentane contenant 300 mg du précurseur Pr de formule générale (3) tel qu'utilisé dans l'exemple 1.

Le mélange ainsi obtenu est maintenu à 25°C pendant 3 heures. Au bout de ce temps, on obtient un composé organo-métallique de tungstène greffé sur la silice-alumine, l'excès de précurseur Pr qui n'a pas réagi, étant éliminé par lavage avec du n-pentane à 25°C. Le composé organo-métallique de tungstène ainsi greffé est séché sous vide. Il contient 1,5 % en poids de tungstène et répond à la formule générale:

(Si-O)ₓ W [-CH₂-C(CH₃)₃]_{y} [=CH-C(CH₃)] (5)

avec x=1 et y=2.

Dans une seconde étape, on isole 50 mg du composé organo-métallique de tungstène greffé obtenu précédemment qui sont soumis dans un réacteur en verre à un traitement d'hydrogénolyse par mise en contact avec de l'hydrogène, sous une pression absolue d'hydrogène de 73 kPa, à 150°C, pendant 15 heures. Au bout de ce temps, on refroidit le réacteur à 25°C, on obtient et on isole sous argon un composé (W/SiAl-1) selon l'invention qui comprend notamment un hydrure de tungstène greffé sur silice-alumine. Le composé (W/SiAl-1) contient 1,5 % en poids de tungstène et présente en spectroscopie infra-rouge deux bandes d'absorption respectivement à 1906 et 1804 cm⁻¹, caractéristiques de la liaison (W-H) notamment greffée sur silice-alumine.

### Exemple 5: préparation d'un hydrure de tungstène greffé sur une silice alumine.

Les étapes préalables de calcination et de déshydroxylation de la silice-alumine sont exactement identiques à celles décrit dans l'exemple 4.

Dans une première étape, on isole 1 g de la silice-alumine préparée précédemment et on les introduit sous atmosphère d'argon dans un réacteur en verre à 25°C muni d'un barreau magnétique d'agitation. Puis dans le réacteur, on introduit 305 mg du précurseur Pr de formule générale (3) tel qu'utilisé à l'Exemple 1. On chauffe le réacteur à 66°C et on agite le mélange à sec ainsi réalisé pendant 4 heures. Au bout de ce temps on refroidit le réacteur à 25°C, puis on lave le mélange de solide avec du n-pentane à 25°C. Le composé solide ainsi lavé est séché sous vide, puis isolé sous argon de façon à obtenir un composé organométallique de tungstène greffé sur la silice-alumine contenant 7,5 % en poids de tungstène et qui répond à la formule générale (5) précédente.

On réalise la seconde étape exactement comme à l'Exemple 1, excepté le fait qu'on utilise le composé organométallique de tungstène greffé sur la silice alumine préparé précédemment. On obtient ainsi un composé (W/SiAl-2) selon l'invention comprenant un hydrure de tungstène greffé sur la silice alumine et contenant 7,5 % en poids de tungstène. Il présente en spectroscopie infra-rouge deux bandes d'absorption respectivement à 1903 et 1804 cm⁻¹, caractéristiques de la liaison (W-H) greffée sur la silice alumine. Par ailleurs, il présente en résonance magnétique nucléaire (¹H-RMN solide) sous 500 MHz une valeur du déplacement chimique de l'hydrure de tungstène (δ_{W-H}) égale à 10,6 ppm (parties par million).

### Exemple 6: Conversion catalytique de l'éthylène en propène sur (W/Al-3) en réacteur statique.

Le composé métallique supporté (W/Al-3), préparé selon l'exemple 3, est utilisé dans une réaction de conversion de l'éthylène en propène qui peut être représentée par l'équation suivante:

3 CH₂= CH₂ → 2 CH₂=CH-CH₃

L'expérience est réalisée de la façon suivante: le composé métallique supporté est préparé «in situ» dans un réacteur en verre comme décrit dans l'exemple 3. Le réacteur est ensuite mis sous vide, puis rempli d'éthylène jusqu'à une pression de 76 kPa, et enfin chauffé à 150°C. On observe alors la formation d'un mélange essentiellement de propène, de n- et iso-butènes, et également d'hexènes en plus faible quantité qui sont analysés et dosés par chromatographie en phase gaz (colonne capillaire KCl/Al₂O₃ 50m x 0,32 mm; détection par ionisation de flamme).

On calcule la conversion cumulée de l'éthylène qui est le nombre de moles d'éthylène transformées sur le nombre de moles d'éthylène introduites initialement ainsi que le nombre de rotations (T.O.N.) ou nombre cumulé de moles d'éthylène transformées au cours du temps par mole de tungstène du composé métallique supporté.

Les sélectivités (SC₃), (SC₄) et (SC₆) en les différents produits sont aussi calculées respectivement selon les équations suivantes:
SC₃ = (nombre de moles de propène formé)/(nombre total de moles d'oléfines formées)
SC₄ = (nombre de moles de butènes formés)/(nombre total de moles d'oléfines formées)
SC₆ = (nombre de moles d'hexènes formés)/(nombre total de moles d'oléfines formées)

Le Tableau 1 ci-après rassemble les résultats des mesures et calculs définis ci-dessus pour la réaction de conversion de l'éthylène en propène en fonction du temps.

| | | | Sélectivités des produits formés % | | |
|---|---|---|---|---|---|
| Temps (h) | Conversion cumulée % | T.O.N. | SC3 Propène % | SC4 Butènes % | SC6 Hexènes % |
| 0 | 0 | 0 | 0 | 0 | 0 |
| 2 | 3,2 | 33 | 82,3 | 16,8 | 0,91 |
| 22 | 23 | 304 | 92,1 | 6,6 | 1,26 |
| 46 | 34 | 348 | 90,55 | 8,18 | 1,28 |
| 76 | 47,5 | 480 | 87,18 | 11,57 | 1,25 |

| | | | | | |
|---|---|---|---|---|---|
| T.O.N = nombre de moles d'éthylène converti par mole de tungstène de surface | | | | | |

L'analyse des résultats du Tableau 1 montre que le composé métallique supporté selon l'invention (W/Al-3) présente une activité catalytique dans la réaction de conversion directe d'éthylène en propène, extrêmement élevée avec une sélectivité élevée en propène autour de 90 %.

### Exemple 7: Conversion catalytique de l'éthylène en propène sur (W/Al-3) en réacteur dynamique.

Le complexe (Al-O)ₓ W [-CH₂-C(CH₃)₃]_{y} [=CH-C(CH₃)] (4) greffé sur l'alumine par sublimation (500mg; 3,9 % W/Al₂O₃; 106,6 micromoles de W) selon l'exemple 3 est transféré en boite à gants dans un réacteur tubulaire en inox pouvant être isolé de l'atmosphère. Après connexion du réacteur au montage, le circuit est purgé à l'argon, puis le catalyseur hydrure de tungstène supporté [W]ₛ-H est préparé in situ par traitement, sous courant d'hydrogène (3 ml/min) à 150°C pendant 15h, des complexes alkyle-alkylidynes greffés, conduisant au composé (W/Al-3). Après refroidissement à 25 °C, le réacteur est purgé à l'argon de l'excès d'hydrogène, puis sous courant d'éthylène à 101,3 kPa (4 ml/min, soit un débit molaire de 1,7 éthylène/W/min). Le réacteur est alors amené rapidement à la température de 150°C (montée de 250°C/h). L'analyse des produits est effectuée en ligne par chromatographie en phase gaz (colonne capillaire KCl/Al₂O₃ 50m x 0,32 mm; détection par ionisation de flamme). On observe alors la formation principalement de propène, de butènes et d'hexènes en faibles quantités.

Le Tableau 2 ci-après rassemble les résultats des mesures et calculs comme définis dans l'exemple 6, pour la réaction de conversion instantannée qui est le nombre de moles d'éthylène transformées sur le nombre de moles d'éthylène introduites à chaque instant.

| | | | Sélectivités des produits formés % | | |
|---|---|---|---|---|---|
| Temps (h) | conversion instantannée | T.O.N. cumulé | Propène % | Butènes | Hexènes |
| 0 | 0 | 0 | 0 | 0 | 0 |
| 0,53 | 3,9 | 17 | 54 | 37 | 0 |
| 1,54 | 30 | 35 | 88 | 8,5 | 0 |
| 1,80 | 25 | 42 | 88,7 | 7,5 | 0 |
| 2,33 | 20 | 54 | 90,2 | 7 | 0 |
| 3,12 | 16,4 | 69 | 91 | 6 | 0 |
| 3,92 | 14,5 | 81,4 | 91,5 | 5,77 | 0 |
| 5,5 | 12,53 | 103 | 91,8 | 5,42 | 0 |
| 8,11 | 11 | 134 | 92,4 | 5,08 | 0,25 |
| 12,67 | 9,4 | 180 | 92,8 | 4,8 | 0,23 |
| 16,6 | 8,6 | 215 | 93 | 4,6 | 0,23 |
| 19,85 | 8,03 | 243 | 93,6 | 4,5 | 0,21 |
| 23,76 | 7,54 | 274 | 93,66 | 4,41 | 0,21 |
| 27,67 | 7,03 | 298 | 93,9 | 4,3 | 0,20 |
| 32,88 | 6,52 | 335 | 94,1 | 4,22 | 0,19 |
| 37,44 | 6,2 | 364 | 94,3 | 4,14 | 0,189 |
| 41,34 | 6 | 389 | 93 | 4 | 0,186 |
| 49,81 | 5,5 | 439 | 94,3 | 4 | 0,184 |
| 55,68 | 5,33 | 472 | 94,5 | 3,99 | 0,167 |
| 62,18 | 5,02 | 506 | 94,66 | 3,95 | 0,166 |
| 68,04 | 4,92 | 536 | 94,7 | 3,94 | 0,139 |
| 75,21 | 4,7 | 572 | 94,77 | 3,89 | 0,138 |
| 82,37 | 4,54 | 606 | 94,87 | 3,85 | 0,135 |
| 88,87 | 4,4 | 635 | 95 | 3,85 | 0,138 |
| 95,39 | 4,47 | 664 | 93 | 3,73 | 0,131 |
| 100,67 | 4,27 | 687 | 94,9 | 3,8 | 0,136 |
| 108,45 | 4,12 | 721 | 95 | 3,78 | 0,136 |
| 114,33 | 4,12 | 746 | 94,9 | 3,7 | 0,109 |
| 117 | 4,08 | 760 | 95 | 3,7 | 0,107 |

| | | | | | |
|---|---|---|---|---|---|
| T.O.N = nombre de moles d'éthylène convertis par mole de tungstène de surface | | | | | |

### Exemple 8: Conversion catalytique de l'éthylène en propène sur (W/Al-Si-2) en réacteur dynamique.

Le complexe (si-O)ₓ, W [-CH₂-C(CH₃)₃]_{y} [=CH-C(CH₃)] (5) greffé sur la silice-alumine par sublimation (250 mg; 7,5 % W / Si0₂-Al₂O₃; 204 micromoles de W) selon l'exemple 5, est transféré en boite à gants dans un réacteur tubulaire en inox pouvant être isolé de l'atmosphère. Après connexion du réacteur au montage, le circuit est purgé à l'argon, puis le catalyseur hydrure de tungstène supporté [W]ₛ-H est préparé in situ par traitement, sous courant d'hydrogène (3 ml/min) à 150°C pendant 15h, des complexes alkyle-alkylidynes greffés, conduisant au composé (W/Al-Si-2). Après refroidissement à 25°C, le réacteur est purgé à l'argon de l'excès d'hydrogène, puis sous courant d'éthylène à 101,3 kPa (4 ml/min, soit un débit molaire de 1,7 éthylène/W/min). Le réacteur est alors amené rapidement à la température de 150°C (montée de 250°C/h). L'analyse des produits est effectuée en ligne par chromatographie en phase gaz (colonne capillaire KCl/Al₂O₃ 50m x 0,32 mm; détection par ionisation de flamme). Comme l'exemple précédent, on observe alors la formation principalement de propène, de butènes et d'hexènes en faibles quantités.

## Revendications

1. Procédé de tranformation de l'éthylène en propylène, dans lequel on fait réagir l'éthylène avec un composé métallique supporté comprenant un support à base d'oxyde d'aluminium sur lequel est greffé un hydrure de tungstène.

2. Procédé selon la revendication 1, dans lequel le degré d'oxydation du tungstène a une valeur choisie dans une gamme allant de 2 à 6, de préférence de 4 à 6.

3. Procédé selon la revendication 1 ou 2, dans lequel l'atome de tungstène est lié à un ou plusieurs atomes d'hydrogène et éventuellement à un ou plusieurs radicaux hydrocarbonés, R.

4. Procédé selon la revendication 3, dans lequel les radicaux hydrocarbonés R sont des radicaux hydrocarbonés, identiques ou différents, saturés ou insaturés, comprenant de 1 à 20, de préférence de 1 à 10 atomes de carbone et comprenant éventuellement du silicium.

5. Procédé selon la revendication 1, dans lequel l'hydrure de tungstène est greffé sur le support à base d'oxyde d'aluminium suivant le schéma suivant : 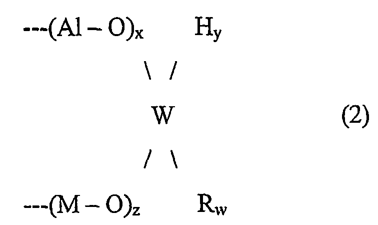 dans lequel W, Al, O et H représentent respectivement des atomes de tungstène, d'aluminium, d'oxygène et d'hydrogène, M représente un atome d'un ou plusieurs éléments d'un autre oxyde, R représente un radical hydrocarboné, et x, y, w et z sont des nombres entiers dont la somme (w + x + y + z) = 2 à 6, et avec x = 1 à 3, y = 1 à 5, w = 0 à 4 et z = 0 à 2, les liaisons ---(A1- O) et ---(M - O) représentant une ou plusieurs liaisons simples ou multiples reliant respectivement l'atome d'aluminium et l'atome M à l'un des constituants atomiques du support à base d'oxyde d'aluminium, notamment à l'un des atomes d'oxygène de ce support.

6. Procédé selon l'une quelconque des revendications 1 à 5, dans lequel le support est choisi parmi les supports homogènes en composition à base d'oxyde d'aluminium et parmi les supports hétérogènes à base d'oxyde d'aluminium comprenant de l'oxyde d'aluminium essentiellement en surface desdits supports.

7. Procédé selon l'une quelconque des revendications 1 à 6, dans lequel le support comprend de l'oxyde d'aluminium, des oxydes mixtes d'aluminium ou des oxydes d'aluminium modifiés.

8. Procédé selon l'une quelconque des revendications précédentes, dans lequel la réaction entre l'éthylène et le composé métallique est conduite à une température allant de 20 à 600 °C, de préférence de 50 à 350 °C, plus préférentiellement de 100 à 250 °C.

9. Procédé selon l'une quelconque des revendications précédentes, dans lequel la réaction entre l'éthylène et le composé métallique est conduite sous une pression absolue allant de 0,01 à 8 MPa, de préférence de 0,01 à 1 MPa, mieux de 0,1 à 0,5 mpA.

10. Procédé selon l'une quelconque des revendications précédentes, dans lequel le composé métallique est régénéré en présence d'hydrogène.

11. Procédé selon.la revendication 10, dans lequel la pression partielle d'hydrogène est comprise entre 0,01 et 10 MPa, de préférence entre 0,1 et 2 MPa.

12. Procédé selon la revendication 10 ou 11, dans lequel la régénération est conduite à une température comprise entre 50 et 300 °C, de préférence entre 80 et 200 °C.

## Claims

1. A process for the conversion of ethylene to propylene, in which ethylene is reacted with a supported metal compound comprising a support based on aluminum oxide to which a tungsten hydride is grafted.

2. The process as claimed in claim 1, in which the degree of oxidation of the tungsten has a value chosen within a range extending from 2 to 6, preferably from 4 to 6.

3. The process as claimed in claim 1 or 2, in which the tungsten atom is bonded to one or more hydrogen atoms and optionally to one or more hydrocarbon radicals R.

4. The process as claimed in claim 3, in which the hydrocarbon radicals R are identical or different, saturated or unsaturated, hydrocarbon radicals comprising from 1 to 20, preferably from 1 to 10, carbon atoms and optionally comprising silicon.

5. The process as claimed in claim 1, in which the tungsten hydride is grafted to the support based on aluminum oxide according to the following scheme: 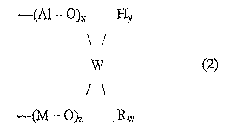 in which W, Al, O and H respectively represent tungsten, aluminum, oxygen and hydrogen atoms, M represents an atom of one or more elements of another oxide, R represents a hydrocarbon radical and x, y, w and z are integers, the sum (w+x+y+z) of which is equal to 2 to 6, and with x = 1 to 3, y = 1 to 5, w = 0 to 4 and z = 0 to 2, the ---(Al-O) and ---(M-O) bonds representing one or more single or multiple bonds respectively connecting the aluminum atom and the atom M to one of the atomic constituents of the support based on aluminum oxide, in particular to one of the oxygen atoms of this support.

6. The process as claimed in any one of claims 1 to 5, in which the support is chosen from supports homogeneous in composition based on aluminum oxide and from heterogeneous supports based on aluminum oxide comprising aluminum oxide essentially at the surface of said supports.

7. The process as claimed in any one of claims 1 to 6, in which the support comprises aluminum oxide, mixed aluminum oxides or modified aluminum oxides.

8. The process as claimed in any one of the preceding claims, in which the reaction between the ethylene and the metal compound is carried out at a temperature ranging from 20 to 600°C, preferably from 50 to 350°C, more preferably from 100 to 250°C.

9. The process as claimed in any one of the preceding claims, in which the reaction between the ethylene and the metal compound is carried out under an absolute pressure ranging from 0.01 to 8 MPa, preferably from 0.01 to 1 MPa, better still from 0.1 to 0.5 MPa.

10. The process as claimed in any one of the preceding claims, in which the metal compound is regenerated in the presence of hydrogen.

11. The process as claimed in claim 10, in which the partial hydrogen pressure is between 0.01 and 10 MPa, preferably between 0.1 and 2 MPa.

12. The process as claimed in claim 10 or 11, in which the regeneration is carried out at a temperature of between 50 and 300°C, preferably between 80 and 200°C.

## Patentansprüche

1. Verfahren zur Umwandlung von Ethylen in Propylen, bei dem man das Ethylen mit einer Träger-Metallverbindung reagieren lässt, die einen Träger auf Aluminiumoxidbasis umfasst, auf den ein Wolframhydrid aufgepfropft ist.

2. Verfahren nach Anspruch 1, bei dem der Oxidationsgrad des Wolframs einen Wert ausgewählt aus dem Bereich 2 bis 6, vorzugsweise 4 bis 6 aufweist.

3. Verfahren nach Anspruch 1 oder 2, bei dem das Wolframatom an ein oder mehrere Wasserstoffatome und gegebenenfalls an ein oder mehrere Kohlenwasserstoffradikale R gebunden ist.

4. Verfahren nach Anspruch 3, bei dem die Kohlenwasserstoffradikale identische oder unterschiedliche, gesättigte oder ungesättigte Kohlenwasserstoffradikale sind, die 1 bis 20, vorzugsweise 1 bis 10 Kohlenstoffatome umfassen und gegebenenfalls Silizium umfassen.

5. Verfahren nach Anspruch 1, bei dem das Wolframhydrid auf den Träger auf Aluminiumoxidbasis aufgepfropft ist nach der folgenden Formel: 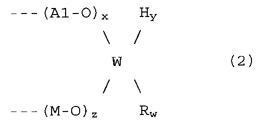 wobei W, Al, O und H jeweils die Wolfram-, Aluminium-, Sauerstoff- und Wasserstoffatome darstellen, M ein Atom eines oder mehrerer Elemente eines anderen Oxids darstellt, R ein Kohlenwasserstoffradikal darstellt und x, y, w und z ganze Zahlen sind, deren Summe (w+x+y+z) = 2 bis 6 und mit x = 1 bis 3, y = 1 bis 5, w = 0 bis 4 und z = 0 bis 2, wobei die Bindungen -(Al-O) und -(M-O) eine oder mehrere Einfachbindungen oder Mehrfachbindungen darstellen, die jeweils das Aluminiumatom und das Atom M an eine der atomaren Bestandteile des Trägers auf Aluminiumoxidbasis, insbesondere an eines der Sauerstoffatome dieses Trägers binden.

6. Verfahren nach einem beliebigen der Ansprüche 1 bis 5, bei dem der Träger ausgewählt ist aus in Mischung homogenen Trägern auf Aluminiumoxidbasis und aus heterogenen Trägern auf Aluminiumoxidbasis, die Aluminiumoxid hauptsächlich an der Oberfläche der Träger aufweisen.

7. Verfahren nach einem beliebigen der Ansprüche 1 bis 6, bei dem der Träger Aluminiumoxid, gemischte Oxide des Aluminiums oder modifizierte Aluminiumoxide umfasst.

8. Verfahren nach einem beliebigen der vorhergehenden Ansprüche, bei dem die Reaktion zwischen dem Ethylen und der Metallverbindung bei einer Temperatur von 20 bis 600°C, vorzugsweise 50 bis 350°C, besonders bevorzugt 100 bis 250°C durchgeführt wird.

9. Verfahren nach einem beliebigen der vorhergehenden Ansprüche, bei dem die Reaktion zwischen dem Ethylen und der Metallverbindung bei einem Absolutdruck von 0,01 bis 8 MPa, vorzugsweise 0,01 bis 1 MPa, besser noch 0,1 bis 0,5 MPa durchgeführt wird.

10. Verfahren nach einem beliebigen der vorhergehenden Ansprüche, bei dem die Metallverbindung in Anwesenheit von Wasserstoff regeneriert wird.

11. Verfahren nach Anspruch 10, bei dem der Partialdruck des Wasserstoffs zwischen 0,01 und 10 MPa, vorzugsweise zwischen 0,1 und 2 MPa liegt.

12. Verfahren nach Anspruch 10 oder 11, bei dem die Regeneration bei einer Temperatur zwischen 50 und 300°C, vorzugsweise zwischen 80 und 200°C, durchgeführt wird.
